# EUROPEAN PATENT SPECIFICATION

(11) **EP 3 518 921 B1**
(45) Date of publication and mention of the grant of the patent: **11.08.2021**
(21) Application number: 17772438.2
(22) Date of filing: 27.09.2017
(51) Int. Cl.: A61K 31/353, A61K 31/025, A61P 27/02

(54) **COMPOSITIONS COMPRISING A CANNABINOID RECEPTOR BINDING LIGAND**
ZUSAMMENSETZUNGEN MIT EINEM CANNABINOIDREZEPTORBINDENDEN LIGANDEN
COMPOSITIONS COMPRENANT UN LIGAND DE LIAISON DE RÉCEPTEUR CANNABINOÏDE

(30) Priority: 28.09.2016 EP 16191194; 26.04.2017 EP 17168172
(43) Date of publication of application: 07.08.2019
(60) Divisional application: 20159519.6
(73) Proprietor: Novaliq GmbH, 69120 Heidelberg (DE)
(72) Inventor: GÜNTHER, Bernhard, 69221 Dossenheim (DE); LÖSCHER, Frank, 69198 Schriesheim (DE); KRÖSSER, Sonja, 69124 Heidelberg (DE); STEVEN, Philipp, 50933 Köln (DE)
(74) Representative: Pharma Patents International AG
(86) International application number: PCT/EP2017/074545
(87) International publication number: WO 2018/060282

(56) References cited:
- GROTENHERMEN F: "Cannabinoids for therapeutic use: Designing systems to increase efficacy and reliability", AMERICAN JOURNAL OF DRUG DELIVERY, vol. 2, no. 4, 1 January 2004 (2004-01-01) , pages 229-240, XP008134461, ADIS INTERNATIONAL, AUCKLAND ; YARDLEY, PA, USA ISSN: 1175-9038, DOI: 10.2165/00137696-200402040-00003
- DUTESCU R M ET AL: "Semifluorinated alkanes as a liquid drug carrier system for topical ocular drug delivery", EUROPEAN JOURNAL OF PHARMACEUTICS AND BIOPHARMACEUTICS, vol. 88, no. 1, 15 May 2014 (2014-05-15), pages 123-128, XP029053290, ADIS INTERNATIONAL, AUCKLAND ; YARDLEY, PA, USA ISSN: 0939-6411, DOI: 10.1016/J.EJPB.2014.05.009
- STEVEN P. ET AL.: "Semifluorinated Alkane Eye Drops for Treatment of Dry-Eye Disease - A Prospective, Multicenter, Non Interventional Study (abstract 4493)", IOVS , vol. 56, no. 7 June 2015 (2015-06), XP002767134, 2015 ARVO Annual Meeting (May 3 - 7, 2015), Denver, Colorado Retrieved from the Internet: URL:http://iovs.arvojournals.org/article.a spx?articleid=2334443&resultClick=1 [retrieved on 2017-02-09]
- GEHLSEN U ET AL.: "Omega-3 Fatty Acids using a F6H8-Carrier as Topical Therapy in Experimental Dry Eye Disease (abstract 417)", IOVS , vol. 57, no. 12 1 May 2016 (2016-05-01), XP002767135, 2016 ARVO Annual Meeting (May 1 - 5, 2016), Seattle, Washington Retrieved from the Internet: URL:http://iovs.arvojournals.org/article.a spx?articleid=2559275&resultClick=1 [retrieved on 2017-02-09]

## Description

### BACKGROUND OF THE INVENTION

Keratoconjunctivitis sicca, also known as dry eye disease or dysfunctional tear syndrome, is today understood as a multifunctional disorder of the tear film and of the ocular surface which results in discomfort, visual disturbance, and often even in ocular surface damage caused by tear film instability. Its prevalence differs widely by regions and is estimated to range from about 7.4% in the USA to about 33% in Japan (J. L. Gayton, Clinical Ophthalmology 2009:3, 405-412). According to another estimate, approximately 3.2 million women and 1.05 million men suffer from keratoconjunctivitis sicca in the USA alone. If symptomatically mild cases are also considered, there could be as many as 20 million affected people in the USA.

The main physiological function of the tear film is the lubrication of the ocular surface and the inner eyelid. In addition, it supplies the ocular surface with the nutrients which it requires, provides a smooth and regular optical surface for the eye. Moreover, it protects the ocular surface against pathogens by various mechanisms, including mechanical removal of foreign particles but also through antimicrobial substances which it contains.

The tear film is composed of a mucous component, an aqueous component, and a lipid component. The inner layer of the film is the mucous layer or component, which is bound to the ocular epithelium via the interaction of mucin molecules which are produced by conjunctival goblet cells and by stratified squameous cells of the conjunctiva and the cornea. The lubricating effect of the tear film is substantially based on the mucous layer and its composition.

On top of the mucous layer is the aqueous layer which is produced by the main and accessory lacrimal glands. Its primary function is to hydrate the mucous component and contribute to the transport of nutrients, electrolytes, antibacterial compounds, and oxygen to the ocular surface. The aqueous component contains water, electrolytes, lysozyme, lactoferrin, immunoglobulins (in particular IgA), retinol, hepatocyte growth factor, epidermal growth factor as its important constituents.

The lipid layer which covers the aqueous layer is produced by the tarsal glands which are positioned at the tarsal plates of the eyelids, and to some degree also by the glands of Zeis which open into the eyelash follicles. Its functions include the enhancement of the spreading of the tear film, decrease of water loss from the aqueous layer by reducing evaporation, and preventing tear film contamination.

It is today acknowledged that keratoconjunctivitis sicca is a complex, multifunctional disorders involving several interacting pathophysiological mechanisms which are only beginning to be understood (H. D. Perry, Am. J. Man. Care 13:3, S79-S87, 2008). The two mechanisms that are being discussed as pivotal in the etiology of the disease and which also appear to reinforce each other mutually are tear hyperosmolarity and tear film instability. Hyperosmolar tear fluid can result from excessive tear film evaporation or reduced aqueous flow. It activates an inflammatory cascade and causes the release of inflammatory mediators into the tear fluid, with multiple pathophysiological effects eventually leading to increased tear film evaporation and tear film instability. Thus, tear film instability can be a consequence of hyperosmolarity. Alternatively, it can develop as the original etiological pathway, e.g. via abnormalities of the lipid layer composition, such as in tarsal gland disease).

Once keratoconjunctivitis sicca has become manifest, inflammation is one of the key processes that maintain and potentially progress the disease. Depending on the severity of the condition, patients often develop a reversible squameous metaphase and punctate erosions of the ocular epithelium. Secondary diseases whose development may be triggered by keratoconjunctivitis sicca include filamentary keratitis, microbial keratitis, corneal neovascularisation, and ocular surface keratinisation.

Two major categories of keratoconjunctivitis sicca or dry eye disease (DED) are distinguished today, which are aqueous-deficient DED and evaporative DED. Within the class of aqueous-deficient forms of DED, two major subtypes are differentiated, Sjögren and non-Sjögren. Sjögren syndrome patients suffer from autoimmune disorders in which the lacrimal glands are invaded by activated T-cells, which leads not only to keratoconjunctivitis sicca but also to a dry mouth condition. The Sjögren syndrome can be a primary disease or result from other autoimmune diseases such as systemic lupus erythrematosus or rheumathroid arthritis. Non-Sjögren patients suffering from an aqueous-deficient DED usually have a lacrimal gland insufficiency, lacrimal duct obstruction or reflex hyposecretion. The second major class, evaporative DED, is also somewhat heterogeneous and can develop as a result of diverse root causes. One of the major causes is meibomian gland disease, eyelid aperture disorders, blink disorders (as in Parkinson disease) or ocular surface disorders (as in allergic conjunctivitis).

Among the many risk factors for keratoconjunctivitis sicca that are known today, some of the best studied ones are advanced age and female sex. It appears that in particular postmenopausal women have a reduced tear production, probably related to hormonal effects which are not very well understood as yet. Further risk factors include diets with low omega-3-fatty acids, occupational factors (e.g. associated with reduced blink frequency), environmental conditions, contact lens wearing, certain systemic (anticholinergics, beta-blockers, isotretinoin, interferons, hormones) and ophthalmic medications (any frequently administered eye drops including artificial tears; especially formulations comprising preservatives), and a number of primary diseases such as Parkinson disease, hepatitis C, HIV infection, and diabetes mellitus.

The management of keratoconjunctivitis sicca relies on both non-pharmacological and pharmacological approaches and the therapeutic options depend significantly on the severity of the disease state (M. A. Lemp, Am. J. Man. Care 14:3, S88-S101, 2008). Non-pharmacological approaches may be used initially when only mild symptoms occur, or as palliative measures to support medical interventions. They include the avoidance of exacerbating factors such as dry air, wind and drafts, tobacco smoke, modification of working habits; eye lid hygiene; tear supplementation, and physical tear retention by punctal plugs or therapeutic contact lenses.

The mainstay of non-pharmacological DED treatment is the use of artificial tears for tear substitution. Most of the available products are designed as lubricants. In addition, they may function as carriers for nutrients and electrolytes (importantly, potassium and bicarbonate), and some products attempt to correct physical parameters such as an increased osmolarity in certain forms of DED. The major functional component of artificial tear compositions is an agent which increases or adjusts the viscosity and which at the same time exhibits lubricant functionality. Common compounds used for this purpose include carboxymethylcellulose and its sodium salt (CMC, carmellose), polyvinyl alcohol, hydroxypropyl methylcellulose (HPMC, hypromellose), hyaluronic acid and its sodium salt, and hydroxypropyl guar gum. However, compositions with a relatively high viscosity, and in particular gel-type formulations, have a tendency to cause visual blurring.

At least in earlier years, multi-dose formulations for ophthalmic administration had to be preserved using a physiologically acceptable preservative in order to reduce the risk of microbial contamination and infection. Most preservatives are however problematic for DED patients in that they have a potential to negatively affect the ocular surface, thus counteracting the therapeutic intent. As an alternative, single-dose containers for the administration of non-preserved formulations were developed. These are however less convenient to handle than the conventional multi-dose bottles.

For moderate to severe forms of keratoconjunctivitis sicca, non-pharmacological approaches are not normally sufficient to manage the symptoms adequately. However, there are presently not many pharmacological therapies available which have proven to be effective and/or which have been authorised by the regulatory agencies.

At least in the USA, the major pharmacological treatment option for moderate to severe keratoconjunctivitis sicca is ciclosporin (i.e. ciclosporin A, also known as cyclosporine A), which is an approved medicine in the form of an ophthalmic emulsion (Restasis®) for increasing "...tear production in patients whose tear production is presumed to be suppressed due to ocular inflammation associated with keratoconjunctivitis sicca." (Restasis prescribing information). According to the evidence that is available, topical ciclosporin is probably disease-modifying rather than only palliative. It acts as an antagonist in various inflammatory processes and cascades. For example, it reduces conjunctival interleukin-6 (IL-6) levels, decreases activated lymphocytes in the conjunctiva, suppresses other conjunctival inflammatory and apoptotic markers, and increases the number of goblet cells in the conjunctiva (Lemp, ditto.). Furthermore, Ciclosporin is an immunosuppressant drug widely used in post-allergenic organ transplant to reduce the activity of the patient's immune system and, so, the risk of organ rejection.

One of the disadvantages of oil-based formulations for ophthalmic administration is that they inherently have a negative impact on vision. Whether used as oily solutions or oil-in-water emulsions, they exhibit a refractive index which differs substantially from that of physiological tear fluid, which leads to visual disturbances and blurring.

Moreover, oil-based formulations do not readily mix with tear fluid to form a homogenous liquid phase. Oily solutions are altogether immiscible with the aqueous tear fluid, and the exact fate of an emulsion mixed with tear fluid in a physiological setting is not completely predictable.

Oil-in-water emulsions of poorly water-soluble drugs like ciclosporin further exhibit the disadvantage that they have a limited drug load capacity. While the active ingredient may have some solubility in the oil phase, this phase is only dispersed in the coherent aqueous phase of the emulsion so that the maximum overall drug concentration in the formulation is very limited.

In contrast to single phase systems such as aqueous or oily solutions, oil-in-water emulsions are also more complex and difficult to manufacture, especially in sterile form. Frequently, emulsions are not readily sterilisable by thermal treatment without negative impact on the physical properties of the emulsion. On the other hand, aseptic processing is complex, costly, and is associated with higher risks of failure, i.e. microbial contamination of the product.

Furthermore, oil-in-water emulsions are like aqueous solutions prone to microbial contamination during use. If they were to be presented in multi-dose containers which are in principle more cost-efficient and convenient for patients than single-use vials, they would have to be preserved in order to ensure their microbiological quality. At the same time, preservatives which can be used in ophthalmic formulations are potentially damaging to the eye, in particular to the ocular surface, and should be avoided in the context of dry eye disease.

Recently, the FDA approved a new eye drop solution to treat signs and symptoms of dry eye disease (DED) under the tradename Xiidra® (50 mg/ml lifitegrast ophthalmic solution, 5% (w/v) in buffered aqueous solution). Lifitegrast inhibits an integrin, lymphocyte function-associated antigen 1 (LFA-1), from binding to intercellular adhesion molecule 1 (ICAM-1). This mechanism down-regulates inflammation mediated by T lymphocytes.

In view of the disadvantages of the above-described methods of treatment of dry eye disease a variety of other compounds have been investigated for their potential usefulness in ophthalmological indications. Accordingly, K.M. Fischer et al. describe in Am. J. Vet. Res. 2013; 74(2): 275-80) the effects of a topically administered delta-9-tetrahydrocannabinol ophthalmic solution on intraocular pressure and aqueous humor flow rate in dogs.

WO 2008/019146 A2 describes aqueous formulations of cannabinoids such as dronabinol, which are stable at room temperature and are suitable - among other modes of delivery - for ophthalmic delivery. Ophthalmic formulations disclosed comprise an effective amount of a cannabinoid dispersed in a pharmaceutically acceptable carrier selected from the group consisting of lanolin, petrolatum, and combinations thereof as well as mineral oil, polyethylene glycol and water aqueous buffer solution.

US 8,222,292 B2 discloses aqueous-based oral cannabinoid (e.g. dronabinol) solutions that are stable at room or refrigerated temperatures. The solutions comprise water, alcohol, a co-solvent such as dehydrated alcohol, ethanol, propanol, isopropanol, propylene glycol, and propylene glycol and a stabilizer such as an antioxidant. Additionally, ophthalmic preparations are disclosed which may also contain a high molecular weight glycol such as PEG400 and pH modifiers as well as tonicity modifying agents.

US 2013/0011484 A1 discloses compositions comprising a cannabinoid receptor binding agent attached to a nanoparticle, such as nanocrystalline cellulose. The drug delivery properties may be modified with functional moieties. Amongst other types of uses, the use of cannabinoid receptor binding agents to treat, ameliorate or alleviate eye disorders is also contemplated, e.g. for treating eye conditions such as dry eye, allergic reactions or injury due to frictional materials and eye surgeries.

US 2015/0045282 A1 discloses delta-9-THC amino acid esters as THC-prodrugs and suppository formulations thereof. In one aspect, topical ophthalmic formulations for effecting ocular bioavailability of delta-9-THC and reducing the intraocular pressure in the treatment of glaucoma are disclosed, comprising the named THC-prodrugs in an acceptable ophthalmic carrier. The topical ophthalmic formulations may be solutions, emulsions, lipid nanoparticulates or matrix films. The solution formulations will typically require solubilizers, such as surfactants which form micellar solutions. Surfactant examples include polyoxyethylene sorbates, polyoxyl hydrogenated castor oils, and alkoxylated fatty acid esters, sorbitan esters.

WO 2015/053829 A1 discloses ophthalmic liquid formulations comprising *Cineraria maritima* component, and at least one of an NSAID, a carnosine and a cannabinoid, such as delta-9-tetrahydrocannabinol, for the treatment and prevention of ophthalmic diseases. The liquid formulations may be a liquid, gel or ointment suspended in a fluid, and may be suitable for use as an eye drop.

WO2015/074137 A1 discloses methods of treatment of ocular inflammation or ocular neuropathic pain in a subject in need thereof, comprising administering a CB2 target agent, a cannabimimetic agent or a combination thereof. The agent may be a cannabinoid, such as a non-psychotropic cannabinoid or a synthetic cannabinoid. The disclosed ocular pharmaceutical formulations comprising a CB2 target agent may further comprise liposomes and further excipients such as phospholipids and cyclodextrins to avoid the use of organic solvents or may be oil-in-water emulsions.

WO 2016/109531 A1 discloses ophthalmic aqueous solutions comprising cannabinoids for the treatment of glaucoma and for symptomatic relief of conjunctival inflammation. An exemplary eye drop solution comprises CBD (Cannabidiol) and CBG (cannabigerol) dissolved in balanced saline solution and further comprising hydroxymethyl cellulose and polysorbate 80. Furthermore, thickeners, a buffer, a pH adjusting agent or a solubilizer may be added to the eye drop solutions.

The present invention is defined in the appended claims. It is an object of the present invention to provide pharmaceutical compositions which are useful in the treatment and prevention of a broad variety of ophthalmic diseases or disorders such as, e.g. dry eye disease. Furthermore, it is an object of the present invention to provide pharmaceutical compositions which are useful for the treatment or prevention of other ophthalmic diseases and disorders which often accompany or concur dry eye disease (DED), such as damage or loss of corneal nerves (also as a result from corneal damage originating from e.g. corneal surgery, LASIK, corneal trauma) or neuropathic pain and which overcome at least one of the limitations or disadvantages associated with prior art formulations.

In a specific aspect, it is an object of the invention to provide an ophthalmic composition which has the capacity to incorporate substantial amounts of at least one poorly water-soluble cannabinoid receptor type 1 and/or 2 binding ligand which might be useful in the treatment and management of the above-listed diseases and disorders, wherein the at least one cannabinoid receptor type 1 and/or 2 binding ligand is delta-9-tetrahydrocannabinol (THC).

THC is poorly soluble in water and is subject to hydrolytic degeneration in aqueous formulations. As such a specific aspect of the present invention, was to provide an ophthalmic composition comprising THC in a non-degrading stabilized formulation with improved solubility.

In yet a further aspect, it is an object of the invention to provide a pharmaceutical kit comprising at least one cannabinoid receptor type 1 and/or 2 binding ligand which does not exhibit one or more of the disadvantages of the prior art, wherein the at least one cannabinoid receptor receptor type 1 and/or 2 binding ligand is THC. Further objects of the invention will become clear on the basis of the following description, examples, and patent claims.

### SUMMARY OF THE INVENTION

The present invention provides a pharmaceutical composition comprising:
a) a therapeutically effective amount of at least one cannabinoid receptor binding ligand, and
b) a liquid vehicle comprising at least one semifluorinated alkane;
wherein the at least one cannabinoid receptor binding ligand is delta-9-tetrahydrocannabinol (THC) or any pharmaceutically acceptable salt thereof.

In a further aspect, the present invention provides the pharmaceutical compositions of the first aspect of the invention for use as a medicament.

In a further aspect, the present invention provides the pharmaceutical compositions of the first aspect of the invention for use in the treatment of neuropathic pain.

In a further aspect, the present invention provides the pharmaceutical compositions of the first aspect of the invention for use in the treatment of keratoconjunctivitis sicca.

In yet a further aspect, the present invention provides a kit comprising the pharmaceutical composition according to the first aspect of the invention, and comprising a container adapted to hold the pharmaceutical composition and a dispensing means adapted for topical administration of the composition to the eye of a patient.

### BRIEF DESCRIPTION OF THE DRAWINGS

Fig. 1: Experimental setup of the desiccating stress dry eye disease model (mouse)
Fig. 2: Results of fluorescein grading obtained following the protocol as described in Fig. 1 as a measure for corneal healing when comparing topical administration of formulations of THC in F4H5 (0.05 %(w/v), 0.01 % (w/v)) as compared to controls (no treatment, vehicle)
Fig. 3: Results of measurement of tear production obtained following the protocol as described in Fig. 1 when comparing topical administration of formulations of THC in F4H5 (0.05 %(w/v), 0.01 % (w/v)) as compared to controls (no treatment, vehicle)
Fig. 4: Assessment of healing of corneal nerve damage obtained following the protocol as described in Fig. 1 when comparing topical administration of formulations of THC in F4H5 (0.05 %(w/v), 0.01 % (w/v)) as compared to controls (no treatment, vehicle) Fig. 5: Experimental setup of the desiccating stress dry eye disease model (mouse)
Fig. 6: Results of fluorescein grading obtained following the protocol as described in Fig. 5 comparing topical administration of formulations of THC in F4H5 (0.01 % (w/v), 0.05 %(w/v), 0.10 %(w/v) and 0.50 %(w/v)) as compared to controls (no treatment, vehicle (F4H5))
Fig. 7: Results of measurement of tear production following the protocol as described in Fig. 5 comparing topical administration of formulations of THC in F4H5 (0.01 %(w/v), 0.05 %(w/v), 0.10 %(w/v) and 0.50 %(w/v)) to controls (no treatment, vehicle (F4H5))
Fig. 8: Head-to-head comparison of measurement of fluorescein staining following the protocol as described in Fig. 5 with topical administration of a formulation THC in F4H5 (0.1 %(w/v) to the FDA-approved DED medication Xiidra® (lifitegrast ophthalmic solution, 50mg/ml) and control (untreated)
Fig. 9: Head-to-head comparison of measurement of tear production following the protocol as described in Fig. 5 with topical administration of formulation THC in F4H5 (0.1 %(w/v) to the FDA-approved DED medication Xiidra® (lifitegrast ophthalmic solution, 50mg/ml) and control (untreated)

### DETAILED DESCRIPTION OF THE INVENTION

In a first aspect, the present invention relates to a pharmaceutical composition comprising:
a) a therapeutically effective amount of at least one cannabinoid receptor binding ligand, and
b) a liquid vehicle comprising at least one semifluorinated alkane, wherein the least one cannabinoid receptor binding ligand is delta-9-tetrahydrocannabinol (THC).

The term "cannabinoid receptor binding ligand" us used in component a) herein can mean any natural or synthetic compound or chemical that has an affinity to a cannabinoid receptor and produces a biological response. A cannabinoid receptor binding ligand as used herein is preferably an agonist to the cannabinoid receptor. Furthermore, cannabinoid receptor binding ligands as used herein may act as an agonist and may bind to the cannabinoid receptor type 1 (CB-1) and/or to the cannabinoid receptor type 2 (CB-2). Preferably, the cannabinoid receptor binding ligands as used herein bind to the cannabinoid receptor type 1 (CB-1) and to the cannabinoid receptor type 2 (CB-2).

Preferred cannabinoid receptor binding ligands according to the present invention are selected from the group consisting of delta-9-tetrahydrocannabinol (THC), Cannabinol, Cannabidiol (CBD), CP55,940, anandamide (arachidonylethanolamid), N-arachidonylethanolamine (AEA), N-arachidonoyl dopamine, 2-arachidonoylglycerol (2-AG), N-palmityl-ethanolamide (PEA), 2-arachidonyl glyceryl ether, Epigallocatechingallat (EGCG), AM-1221(1-[(N-methylpiperidin-2-yl)methyl]-2-methyl-3-(naphthalen-1-oyl)-6-nitroindole) , AM-1235 (1-[(5-fluoropentyl)-6-nitro-1H-indol-3-yl]-(naphthalen-1-yl)methanone), AM-2232 (5-(3-(1-naphthoyl)-1H-indol-1-yl)pentanenitrile), UR-144 ((1-pentylindol-3-yl)-(2,2,3,3-tetramethylcyclopropyl)methanone), JWH-007 (1-pentyl-2-methyl-3-(1-naphthoyl)indole), JWH-015 ((2-Methyl-1-propyl-1H-indol-3-yl)-1-naphthalenylmethanone), JWH-018 (1-Pentyl-3-naphthoylindol), WIN 55,212-2 ((R)-(+)-[2,3-Dihydro-5-methyl-3-(4-morpholinylmethyl)pyrrolo[1,2,3-de]-1,4-benzoxazin-6-yl]-1-naphthalenylmethanone mesylate), JWH-133 ((6aR,10aR)-3-(1,1-Dimethylbutyl)-6a,7,10,10a-tetrahydro -6,6,9-trimethyl-6H-dibenzo[b,d]pyran), HU-210 ((6aR,10aR)-9-(hydroxymethyl)-6,6-dimethyl-3-(2-methyloctan-2-yl)-6H,6aH,7H,10H,10aH-benzo[c]isochromen-1-ol) and HU-308 ([(1R,2R,5R)-2-[2,6-Dimethoxy-4-(2-methyloctan-2-yl)phenyl]-7,7-dimethyl-4-bicyclo[3.1.1]hept-3-enyl]methanol), EGC (Epigallocatechin), ECG, N-Alkylamide, beta-caryophyllene, falcarinol, rutamarin, 3,3'-diindolylmethane, N-arachidonyl glycine (NAGly), abnormal cannabidiol and HU-308 or pharmaceutically acceptable salts thereof.

In a preferred embodiment of the present invention the at least one cannabinoid receptor binding ligand binds to the cannabinoid receptor type 1 (CB-1) and is selected from the group consisting of delta-9-tetrahydrocannabinol (THC), Cannabinol, Cannabidiol (CBD), CP 55,940, anandamide, N-arachidonoyl dopamine, 2-arachidonoylglycerol, 2-arachidonyl glyceryl ether, EGCG, AM-1221, AM-1235, AM-2232, UR-144, JWH-007, JWH-015, JWH-018, WIN 55,212-2, HU-210 and N-arachidonylethanolamine.

In another preferred embodiment of the present invention the at least one cannabinoid receptor binding ligand binds to the cannabinoid receptor type 2 (CB-2) and is selected from the group consisting of delta-9-tetrahydrocannabinol (THC), Cannabinol, Cannabidiol (CBD), CP 55,940, anandamide, N-arachidonoyl dopamine, 2-arachidonoylglycerol, 2-arachidonyl glyceryl ether, EGCG, EGC, ECG, N-alkylamide, β-caryophyllene, falcarinol, rutamarin, 3,3'-diindolylmethane, AM-1221, AM-1235, AM-2232, UR-144, JWH-007, JWH-015, JWH-018, JWH-133 and HU-308.

In another preferred embodiment of the present invention the at least one cannabinoid receptor binding ligand is selected from the group consisting of delta-9-tetrahydrocannabinol (THC), Cannabidiol (CBD), β-caryophyllene, CP55,940, WIN55,212-2, HU-308 and N-arachidonyl glycine or any pharmaceutically acceptable isomer, derivative, solvate or salt thereof; or selected from any combinations of two or more of the named cannabinoid receptor binding ligands.

In a further preferred embodiment of the present invention the at least one cannabinoid receptor binding ligand is selected from the group consisting of delta-9-tetrahydrocannabinol (THC), Cannabidiol (CBD), β-caryophyllene and HU-308, or selected from the group consisting of delta-9-tetrahydrocannabinol (THC), Cannabidiol (CBD) and β-caryophyllene, or selected from the group consisting of delta-9-tetrahydrocannabinol (THC) and Cannabidiol (CBD), or any pharmaceutically acceptable isomer, derivative, solvate or salt thereof.

In another preferred embodiment of the present invention the at least one cannabinoid receptor binding ligand is selected from the group consisting delta-9-tetrahydrocannabinol (THC), Cannabidiol (CBD) and HU-308 or selected from the group consisting of delta-9-tetrahydrocannabinol (THC) and HU-308 or selected from the group consisting of delta-9-tetrahydrocannabinol (THC), β-caryophyllene and HU-308 or selected from the group consisting of delta-9-tetrahydrocannabinol (THC) and β -caryophyllene; or any pharmaceutically acceptable isomer, derivative, solvate or salt thereof.

In another preferred embodiment of the present invention the at least one cannabinoid receptor binding ligand is selected from the group consisting delta-9-tetrahydrocannabinol (THC) and CP55,940 or selected from the group consisting of delta-9-tetrahydrocannabinol (THC) and WIN55,212-2 or selected from the group consisting of delta-9-tetrahydrocannabinol (THC) and N-arachidonyl glycine; or any pharmaceutically acceptable isomer, derivative, solvate or salt thereof.

In a particularly preferred embodiment the at least one cannabinoid receptor binding ligand is delta-9-tetrahydrocannabinol (THC; (-)-(6a*R*,10a*R*)-6,6,9-Trimethyl-3-pentyl-6a,7,8,10a-tetrahydro-6*H*-benzo[*c*]chromen-1-ol, Dronabinol) or any pharmaceutically acceptable salt thereof.

The term "at least one" as used herein means that only one cannabinoid receptor binding ligands as described above may be used in the pharmaceutical compositions according to the present invention or that a mixture of two or more cannabinoid receptor binding ligands may be used in the same compound. Preferably, one or two different cannabinoid receptor binding ligands, more preferably only one receptor binding ligand is used in the pharmaceutical compositions according to the present invention.

The term "therapeutically effective amount" refers to a dose, concentration or strength which is useful for producing a desired pharmacological effect. In one embodiment, the concentration of each chosen cannabinoid receptor binding ligand, preferably of delta-9-tetrahydrocannabinol (THC), in the pharmaceutical composition according to the present invention may be chosen in the range from about 0,01 to about 10 mg/ml, corresponding to about 0,001 to about 1,0 % (w/v) of the final composition ready for administration (final dosage form).

In a preferred embodiment, the concentration of each chosen cannabinoid receptor binding ligand in the pharmaceutical composition according to the present invention may be chosen in the range from about 0,05 to about 2 mg/ml, corresponding to about 0,005 to about 0,2 % (w/v) of the final composition ready for administration (final dosage form). In an even more preferred embodiment the pharmaceutical composition according to the present invention the concentration of the cannabinoid receptor binding ligand in the pharmaceutical composition may be chosen in the range from about from 0,1 to about 1,0 mg/ml, corresponding to about 0,01 to about 0,1 % (w/v) of the final composition (final dosage form).

The term "semifluorinated alkane" (also referred to as "SFA" throughout this document) as used herein refers to a linear or branched compound composed of at least one perfluorinated segment (F-segment) and at least one non-fluorinated hydrocarbon segment (H-segment). More preferably, the semifluorinated alkane is a linear or branched compound composed of one perfluorinated segment (F-segment) and one non-fluorinated hydrocarbon segment (H-segment). Preferably, said semifluorinated alkane is a compound that exists in a liquid state at least at one temperature within the temperature range of 4° to 40°C. In one embodiment, the perfluorinated segment and/or the hydrocarbon segment of the said SFA optionally comprises or consists of a cyclic hydrocarbon segment, or optionally said SFA comprises an unsaturated moiety within the hydrocarbon segment.

Preferably, the F-segment of a linear or branched SFA comprises between 3 to 10 carbon atoms. It is also preferred that the H-segment comprises between 3 to 10 carbon atoms. It is particularly preferred that the F- and the H-segment comprise, but independently from one another, 3 to 10 carbon atoms. Preferably, each segment independently from another is having 3 to 10 carbon atoms.

It is further preferred, that the F-segment of a linear or branched SFA comprises between 4 to 10 carbon atoms and/or that the H-segment comprises between 4 to 10 carbon atoms. It is particularly further preferred that the F- and the H-segment comprise, but independently from one another, 4 to 10 carbon atoms. Preferably, each segment is independently from another having 4 to 10 carbon atoms.

Accordingly, in a preferred embodiment the liquid vehicle according to component b) of the present invention comprises at least one semifluorinated alkane which is a compound, preferably a linear compound of the formula F(CF₂)ₙ(CH₂)ₘH, wherein n and m are integers independently selected from the range of 3 to 10, preferably selected from the range of 4 to 10 and even more preferably selected from the range of 4 to 8 carbon atoms.

Optionally, the linear or branched SFA may comprise a branched non-fluorinated hydrocarbon segment comprising one or more alkyl groups selected from the group consisting of -CH₃, -C₂H₅, -C₃H₇ and -C₄H₉ and/or the linear or branched SFA may comprise a branched perfluorinated hydrocarbon segment, comprising one or more perfluorinated alkyl groups selected from the group consisting of -CF₃, -C₂F₅, -C₃F₇ and -C₄F₉. It is further preferred that the ratio of the carbon atoms of the F-segment and the H-segment (said ratio obtained by dividing the number of carbon atoms in the F-segment by the numbers of carbon atoms in the H-segment; e.g. said ratio is 0.75 for 1-perfluorohexyloctane (F6H8)) of a linear or branched SFA is ≥0.5, more preferably said ratio is ≥0.6. It is further preferred that the ratio of the carbon atoms of the F-segment and the H-segment is in the range between 0.6 and 3.0, more preferably said ratio is between 0.6 and 1.0.

In a preferred embodiment of the present invention the semifluorinated alkane refers to a linear compound composed of at least one perfluorinated segment (F-segment) and at least one hydrocarbon segment (H-segment). More preferably, said semifluorinated alkane is a linear compound composed of one perfluorinated segment (F-segment) and one hydrocarbon segment (H-segment).

Preferably, the F-segment of a linear SFA comprises between 3 to 10 carbon atoms. It is also preferred that the H-segment comprises between 3 to 10 carbon atoms. It is particularly preferred that the F- and the H-segment comprise, but independently from one another, 3 to 10 carbon atoms. Preferably, each segment independently from another is having 3 to 10 carbon atoms.

It is further preferred, that the F-segment of a linear SFA comprises between 4 to 10 carbon atoms and/or that the H-segment comprises between 4 to 10 and even more preferably 4 to 8 carbon atoms. It is particularly further preferred that the F- and the H-segment comprise, but independently from one another, 4 to 10 carbon atoms, even more preferably 4 to 8 carbon atoms. Preferably, each segment is independently from another having 4 to 10, preferably 4 to 8 carbon atoms.

According to another nomenclature, the linear semifluorinated alkanes as used in the present invention may be referred to as FnHm, wherein F means the perfluorinated hydrocarbon segment, H means the non-fluorinated hydrocarbon segment and n, m is the number of carbon atoms of the respective segment. For example, F4H5 is used for 1-perfluorobutyl pentane.

In a particularly preferred embodiment, the liquid vehicle of the pharmaceutical composition according to component b) of the present invention comprises at least one linear semifluorinated alkane selected from the group consisting of: F4H4, F4H5, F4H6, F4H7, F4H8, F5H4, F5H5, F5H6, F5H7, F5H8, F6H2, F6H4, F6H6, F6H7, F6H8, F6H9, F6H10, F6H12, F8H8, F8H10, F8H12 and F10H10.

More preferably the liquid vehicle of pharmaceutical composition according to component b) of the present invention comprises at least one linear semifluorinated alkane selected from the group consisting of: F4H4, F4H5, F4H6, F5H4, F5H5, F5H6, F5H7, F5H8, F6H2, F6H4, F6H6, F6H7, F6H8, F6H9, F6H10, F8H8, F8H10, F8H12 and F10H10, even more preferably the linear SFA is selected from the group consisting of: F4H4, F4H5, F4H6, F5H4, F5H5, F5H6, F5H7, F5H8, F6H4, F6H6, F6H7, F6H8, F6H9, F6H10, F8H8, F8H10, F8H12 and F10H10, more preferably the linear SFA is selected from the group consisting of: F4H4, F4H5, F4H6, F5H5, F5H6, F5H7, F5H8, F6H6, F6H7, F6H8, F6H9, F6H10, F8H8, F8H10, F8H12 and F10H10 and even more preferably the linear SFA is selected from the group consisting of: F4H4, F4H5, F4H6, F5H5, F5H6, F5H7, F5H8, F6H6, F6H7, F6H8, F6H9, F6H10 and F8H8. In a further preferred embodiment, the liquid vehicle of the pharmaceutical composition according to component b) of the present invention comprises at least one linear SFA selected from the group consisting of: F4H5, F4H6, F5H6, F5H7, F6H6, F6H7 and F6H8. In an even further preferred embodiment the liquid vehicle according to component b) of the present invention comprises at least one linear SFA, preferably at least one linear SFA selected from F4H5 and F6H8. Most preferably the liquid vehicle according to component b) of the present pharmaceutical composition comprises F4H5 (1-perfluorobutyl-pentane) as the only semifluorinated alkane.

In a further embodiment, the semifluorinated alkane of component b) of the present invention may be used as a mixture of two or more different semifluorinated alkanes. Accordingly, the present pharmaceutical compositions may comprise more than one SFA. It may be useful to combine different SFA's, for example, in order to achieve a particular target property such as a certain density or viscosity. If a mixture of two or more different SFA's is used, it is furthermore preferred that the mixture comprises at least one of F4H5, F4H6, F6H4, F6H6, F6H8 and F6H10, and in particular one of F4H5, F6H6 and F6H8. In another embodiment, the mixture comprises at least two members selected from F4H5, F4H6, F6H4, F6H6, F6H8, and F6H10, and in particular at least two members selected from F4H5, F6H6 and F6H8.

Liquid SFA's are chemically and physiologically inert, colourless and stable. Their typical densities range from 1.1 to 1.7 g/cm³, and their surface tension may be as low as 19 mN/m. SFA's of the FnHm type are insoluble in water but also somewhat amphiphilic, with increasing lipophilicity correlating with an increasing size of the non-fluorinated segment.

A particular advantage of liquid SFAs as vehicle, is that in particular compounds such as delta-9-tetrahydrocannabinol (THC), which are prone to hydrolytic degeneration, can be stabilized and protected from degradation.

In another embodiment, the liquid vehicle according to component b) of the present pharmaceutical composition comprises at least one semifluorinated alkane that is liquid at room temperature, such as F4H4, F4H5, F4H6, F5H5, F5H6, F5H7, F5H8, F6H6, F6H7, F6H8, F6H9, F6H10 and F8H8.

The pharmaceutical compositions according to the present invention comprise a therapeutically effective amount of at least one cannabinoid receptor binding ligand or any pharmaceutically acceptable isomer, derivative or salt thereof as component a), and a liquid vehicle comprising at least one semifluorinated alkane as component b). In one embodiment, the pharmaceutical composition according to the present invention consists of at least 75% (w/v) of the at least one semifluorinated alkane, based on the volume of the final composition (final dosage form) comprising the at least one cannabinoid receptor binding ligand and optionally further solvents and excipients.

In a preferred embodiment, the pharmaceutical composition according to the present invention consists of from about 80% to about 99,9% (w/v), more preferably from about 90% to about 99,5% (w/v) and most preferred from about 95% to about 99% (w/v) of the at least one semifluorinated alkane, based on the volume of the final composition comprising the at least one cannabinoid receptor binding ligand and optionally further solvents and excipients.

Depending on the cannabinoid receptor binding ligand chosen, the pharmaceutical compositions according to the present invention may be in the form of a suspension or in the form of a solution. Preferably, the present pharmaceutical composition is a solution, even more preferably a clear solution.

A clear solution, as understood herein, refers to a liquid solution in which all solutes are fully dissolvable or dissolved under room temperature conditions i.e. between 15-25 °C. The clear solution does not comprise of any particulate or solid phase components and preferably has a refractive index approximate to that of water (i.e. 1.333) at room temperature.

The pharmaceutical compositions of the present invention may as an optional and additional component c) comprise one or more further excipients. The term "excipients" as used herein refers to any pharmaceutically acceptable natural or synthetic substance that may be added to the pharmaceutical compositions of the present invention to enhance or otherwise modify its physical or chemical constitution or stability or therapeutic properties. The pharmaceutical compositions may optionally comprise one or more excipients such as, for example, an antioxidant, a preservative, a lipid or oily excipient, a surfactant or a lubricant or a combination of at least 2 excipients thereof.

Suitable antioxidants for use in the present pharmaceutical compositions comprise, for example: butylated hydroxytoluene (BHT), butylated hydroxyanisole (BHA), tertiary butylhydroquinone (TBHQ), vitamin E, vitamin E derivatives (i.e. alpha-tocopherol acetate) and/or ascorbic acid.

Suitable lipid or oily excipients for use in the present invention comprise, for example, triglyceride oils (i.e. soybean oil, olive oil, sesame oil, cotton seed oil, castor oil, sweet almond oil), triglycerides, mineral oil (i.e. petrolatum and liquid paraffin), medium chain triglycerides (MCT), oily fatty acids, isopropyl myristate, oily fatty alcohols, esters of sorbitol and fatty acids, oily sucrose esters, or any other oily substance which is physiologically tolerated by the eye.

Suitable lubricants for use in the present invention comprise, for example, carboxymethylcellulose and its sodium salt (CMC, carmellose), polyvinyl alcohol, hydroxypropyl methylcellulose (HPMC, hypromellose), hyaluronic acid and its sodium salt, and hydroxypropyl guar gum.

The pharmaceutical composition according to the present invention may or may not comprise pharmaceutically suitable natural or synthetic preservatives, such as, for example, benzalkonium chloride and chlorhexidine. In a preferred embodiment, however, the pharmaceutical compositions according to the present invention do not comprise a pharmaceutically acceptable preservative.

In addition to the excipients as optional components c) as described above the liquid vehicle according to component b) of the present pharmaceutical compositions may also comprise one or more further solvents. The term "further solvents" as used herein refers to a solvent or mixture of two or more different solvents other than the at least one semifluorinated alkane of the liquid vehicle according to component b). Suitable further solvent may be chosen from, for example, alcohols, such as ethanol, isopropanol or other further solvent which is physiologically tolerated by the eye.

A preferred solvent is ethanol which may be present in the present pharmaceutical compositions in an amount of about 1,4 wt.-% or less, preferably up to about 1,0 wt.-%, based on the weight of the final composition (final dosage form).

In a further embodiment, water can also be present in the pharmaceutical compositions of the present invention, however, preferably in small or trace amounts of up 1,0 wt.-% or even up to 0,1 wt.-% or less, based on the final composition (final dosage form). In a preferred embodiment, the pharmaceutical composition of the present invention is essentially free of water, whereas the residual water may be attributed to the potential water content of the chosen cannabinoid receptor binding ligand. The term 'essentially' as used herein means if present then in trace or residual amounts such as to confer no technical advantage or relevance in respect of the object of the invention.

In a particularly preferred embodiment the pharmaceutical compositions of the present invention comprise delta-9-Tetrahydrocannabinol (THC) and/or Cannabidiol and/or β-caryophyllene and/or CP55,940 and/or WIN55,212-2 and/or HU-308 and/or N-arachidonyl glycine, preferably delta-9-Tetrahydrocannabinol (THC) as described above as the at least one cannabinoid receptor binding ligand of component a). The liquid vehicle according to component b) of the present invention comprises preferably semifluorinated alkanes with a high solubility for THC such as, for example F4H5 (1-perfluorobuty-pentane) or F6H8 (1-perfluorohexyl-octane), most preferably F4H5. The pharmaceutical compositions comprising THC dissolved in an semifluorinated alkane, preferably F4H5 as a liquid vehicle may also comprise ethanol as a cosolvent, preferably in an amount of up to 1,4 wt.-%, more preferably in an amount of up to 1,0 wt.% of the final composition (final dosage form). Preferably, the liquid vehicle according to component b) of the pharmaceutical compositions according to this embodiment essentially consists of the at least one, preferably of one semifluorinated alkane or a mixture of different, preferably two different, semifluorinated alkanes and does not contain an additional preservative and/or is essentially free of water.

It was surprisingly found that potentially oxidation-sensitive cannabinoid receptor binding ligands such as, for example, delta-9-Tetrahydrocannabinol (THC) and/or Cannabidiol and/or CP55,940, and/or WIN55,212-2 and/or HU-308 and/or N-arachidonyl glycine, especially delta-9-Tetrahydrocannabinol (THC) can be stored over prolonged periods of time, for example for up to 6 months or even up to 12 months or even up to 2 years when dissolved or suspended in a liquid vehicle according to the present invention comprising at least one semifluorinated alkane. Furthermore, it was surprisingly found that the above-named potentially oxidation sensitive cannabinoid receptor binding ligands, especially delta-9-Tetrahydrocannabinol (THC) were stable even when air was not excluded, especially in cases in which the liquid vehicle had a high content of an semifluorinated alkane such as F4H5 or F6H8 or, most pronounced, when the liquid vehicle essentially consisted of an semifluorinated alkane such as F4H5 or F6H8.

Accordingly, in yet a further preferred embodiment, the pharmaceutical compositions of the present invention may not require the presence of an antioxidant. In a preferred embodiment, the pharmaceutical compositions of the present invention are essentially free of an antioxidant.

Preferably the pharmaceutical compositions of the present invention may consist of a cannabinoid receptor binding ligand, a semifluorinated alkane, optionally a cosolvent such as ethanol and optianally traces of water. More preferably, the pharmaceutical compositions may consist of a cannabinoid receptor binding ligand and a semifluorinated alkane selected from F4H5 (1-perfluorobuty-pentane) or F6H8 (1-perfluorohexyl-octane), a cosolvent such as ethanol and traces of water. Even more preferably the pharmaceutical compositions of the present invention may consist of delta-9-Tetrahydrocannabinol (THC) and a semifluorinated alkane selected from F4H5 (1-perfluorobuty-pentane) or F6H8 (1-perfluorohexyl-octane), most preferably the pharmaceutical compositions may consist of THC and F4H5 (1-perfluorobuty-pentane) and optionally traces of water.

In a further aspect, the present invention is also directed to the use of the pharmaceutical compositions according to the present invention as a medicament.

The pharmaceutical composition according to the present invention are especially useful as ophthalmic compositions, and may preferably be administered topically to the eye, eye sac, eye surface, the cornea, the and/or to an ophthalmic tissue of a patient.

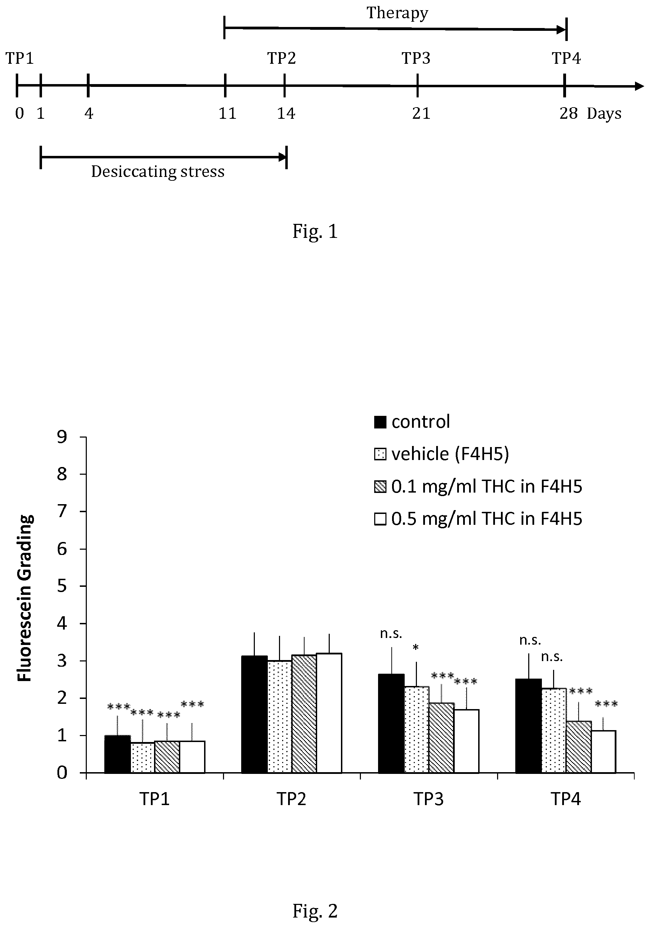

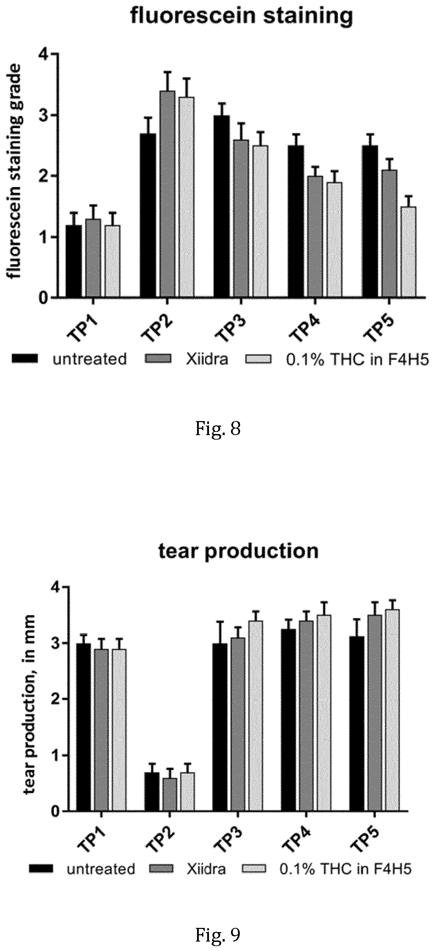

It was surprisingly found that the compositions of the present invention are effective in treatment of ophthalmic diseases or disorders such as in the treatment of neuropathic pain (Fig. 2-4, Fig. 6-9). Further, it was surprisingly found that compositions of the present invention, such as compositions comprising THC and F4H5, show improved effectiveness in healing ocular surface damage (i.e. corneal damage) and in restoring tear production when compared to the recently FDA-approved DED medication Xiidra® although lower concentrations of the active ingredient were employed, as demonstrated by measurement of corneal staining (fluorescein staining) and tear production (Fig. 8-9).

In a further aspect, the present invention relates to the pharmaceutical compositions as described above for use in the treatment of ophthalmic diseases or disorders such as in the treatment neuropathic pain. Further, the present invention also relates to pharmaceutical compositions as described above for use in increasing the tear production in the eye of a subject.

Herein, the need for restoring of a loss or of an impaired corneal sensitivity, may be caused by or related to certain conditions, such as keratoconjunctivitis sicca.

In addition to that, the present invention also relates to the method of using the pharmaceutical compositions of the present invention for the treatment of neuropathic pain. Further, the present invention also relates to the method of using the pharmaceutical compositions of the present invention for increasing the tear production in the eye of a subject.

In yet a further aspect, the present invention relates to the pharmaceutical compositions as described above for use in the treatment of keratoconjunctivitis sicca or a symptom or a condition related thereto. In addition to that, the present invention also relates to the method of using the pharmaceutical compositions of the present invention for the treatment of keratoconjunctivitis sicca.

The pharmaceutical compositions, preferably the ophthalmic compositions of the present invention, therefore offer the unique possibility to provide a combined treatment for keratoconjunctivitis sicca in concurrence (concomitantly) with other ophthalmic diseases and disorders such as neuropathic pain.

In yet a further aspect, the present invention relates to a pharmaceutical kit comprising
i. a pharmaceutical composition comprising a therapeutically effective amount of at least one cannabinoid receptor binding ligand or any pharmaceutically acceptable salt thereof, and a liquid vehicle comprising at least one semifluorinated alkane;
ii. a container for holding the composition, wherein said container comprises a dispensing means adapted for topical administration of the composition to an eye surface, into a lower eyelid, to the lacrimal sac or to an ophthalmic tissue, and
iii. directions for use of the composition in the therapy, treatment, prevention or amelioration of ophthalmic disorders or diseases, wherein the at least one cannabinoid receptor binding ligand is THC.

According to item i) of this aspect of the invention the pharmaceutical kit comprises a pharmaceutical composition as described above for the first aspect of the present invention.

A container as used in connection with item ii) of this aspect of the invention can be provided in any suitable form as a container for single use holding a single dose of the pharmaceutical composition or as a container for multiple uses holding a plurality of single doses. Preferably, the container comprises a dispensing means which allows for dropwise topical administration of the pharmaceutical composition to a surface of the eye of a patient. In one embodiment, the container comprising a dispensing means may be a conventional dropper bottle such as a bottle made of glass or a thermoplastic elastomer with a suitable dispensing means or single-use droppers.

In a further preferred embodiment of this aspect of the invention, the dispensing means comprises a dropper of dimensions such as to dispense droplets having a volume of about 8 to 15 µl, preferably of about 10 µl. With a small droplet volume, precise dosing to the eye (avoiding over-dosing) can be achieved and an excess amount of discharge of a substantial fraction of the composition from the eye subsequent to administration can be avoided.

Directions for use of the pharmaceutical composition according to item iii) of this aspect of the invention can be provided in any suitable form such as, for example, as an enclosed label or instruction leaflet in printed or other readable form. Alternatively, the directions for use can be provided in electronic or computer readable form, such as a barcode or a QR-code.

The directions according to item iii) can comprise instructions for use of the present pharmaceutical compositions in the therapy, treatment, prevention or amelioration of ophthalmic disorders or diseases, preferably in the therapy of inflammation of the cornea and/or the conjunctiva, corneal nerve damage, neuropathic pain or a combination thereof, and/or for use in the therapy of keratoconjunctivitis sicca or a symptom or a condition related thereto.

### DESCRIPTION OF THE DRAWINGS

Fig. 1 shows the experimental setup of the desiccating stress dry eye disease model as described by Yeh, S. et al., Invest. Ophthalmol. Vis. Sci. 44: 124-12 *and* B. Strong, et al. in Cornea Jan 2005;24(1):80-5. Herein, the mice were subjected to desiccating stress from day 1 to day 14. Treatment was administered in form of instillation of 5µl of the respective formulations into the eye (3 times per day) starting at day 11 and continuing to day 28. Tear film production was assessed at TP1 (day 0, healthy mice, before application of desiccating stress), TP2 (day 14, mice at desiccating stress, before therapy), TP3 (day 21, mice after desiccating stress held in standard housing under therapy) and TP4 (day 28, mice after desiccating stress in standard housing under therapy).

Fig. 2 shows the results of fluorescein grading (absolute data; see also Table 2). The separate sets of columns summarize data obtained at TP1 to TP4 for control (no treatment; left column), F4H5 (1-Perfluorobutyl-pentane, second column from the left), 0.1 mg/ml (0.01 %(w/v)) THC in F4H5 (third column from the left) and 0.5 mg/ml (0.05 %(w/v)) THC in F4H5 (right column). Fluorescein data show that in comparison to the control group the formulations of THC in F4H5 lead to the lowest fluorescein staining levels. Therefore, treatment with the THC-formulation with 0.05% (w/v) THC in F4H5 (right column) reveals the highest degree of corneal healing, resulting in fluorescein staining levels at TP4 similar to those recorded at TP1 from healthy animals. (the asterisks refer to the statistical level of significance (p-value) with (*) designating p<0,05, (**) designating p<0,01 and (***) designating p<0,001 when time points TP1, TP3 or TP4 were compared to time point TP2)

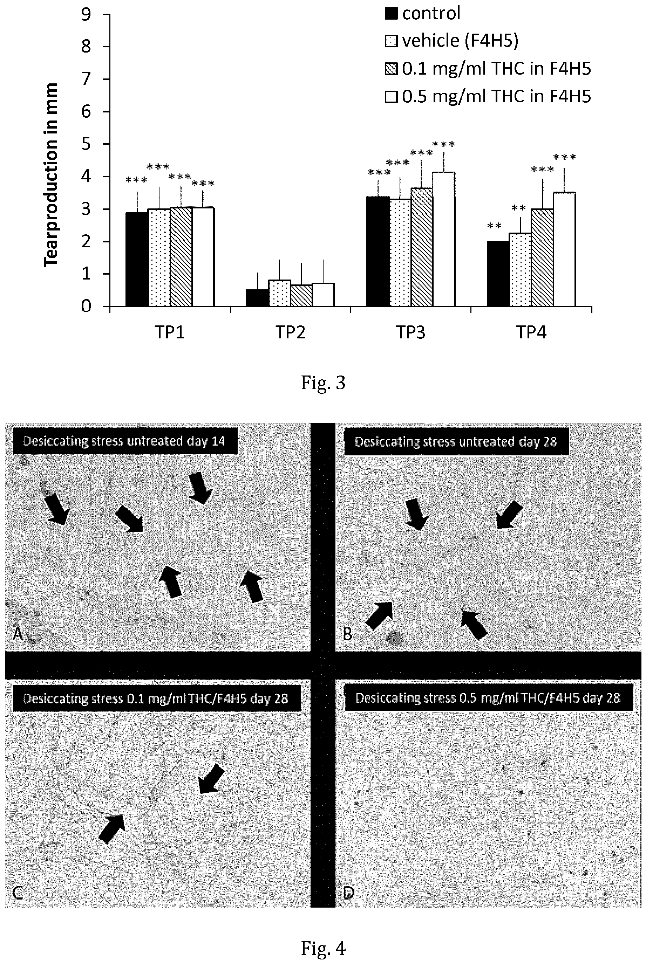

Fig. 3 shows tear production in mm (absolute data; see also Table 1). The separate sets of columns summarize data obtained at TP1 to TP4 for control (no treatment; left column), F4H5 (1-Perfluorobutyl-pentane, second column from the left), 0.1 mg/ml (0.01 %(w/v)) THC in F4H5 (third column from the left) and 0.5 mg/ml (0.05 %(w/v)) THC in F4H5 (right column). The data shows that tear production employing formulations of THC in F4H5 at TP4 are almost raised back to levels previously recorded for healthy animals at TP1, with 0.5 mg/ml (0.05 %(w/v)) THC in F4H5 (right column) resulting in the highest level of tear production. (the asterisks refer to the statistical level of significance (p-value) with (*) designating p<0,05, (**) designating p<0,01 and (***) designating p<0,001 when time points TP1, TP3 or TP4 were compared to time point TP2)

Fig. 4 shows Corneal nerves (subbasal nerve plexus) stained with β-III tubulin as an inverted and sharpened reproduction of the original fluorescence microscopic picture. Picture A on the top left shows loss of corneal nerves (area marked by arrows) at day 14 following desiccating stress. Picture B on the top right shows loss of corneal nerves (arrows) at day 28, following 14 days of desiccating stress and 14 days of recovery period without therapy. Picture C on bottom left shows corneal nerves at day 28, following 14 days of desiccating stress and 14 days of treatment with 0.01 % (w/v) THC in F4H5 (arrows mark the area of corneal nerve loss being substantially smaller than in controls shown in Picture A and B). Picture D on bottom right shows corneal nerves at day 28, following 14 days of desiccating stress and 14 days of treatment with 0.05 % (w/v) THC in F4H5 (no loss of corneal nerves visible, thus indicating highly effective healing of corneal nerve damage).

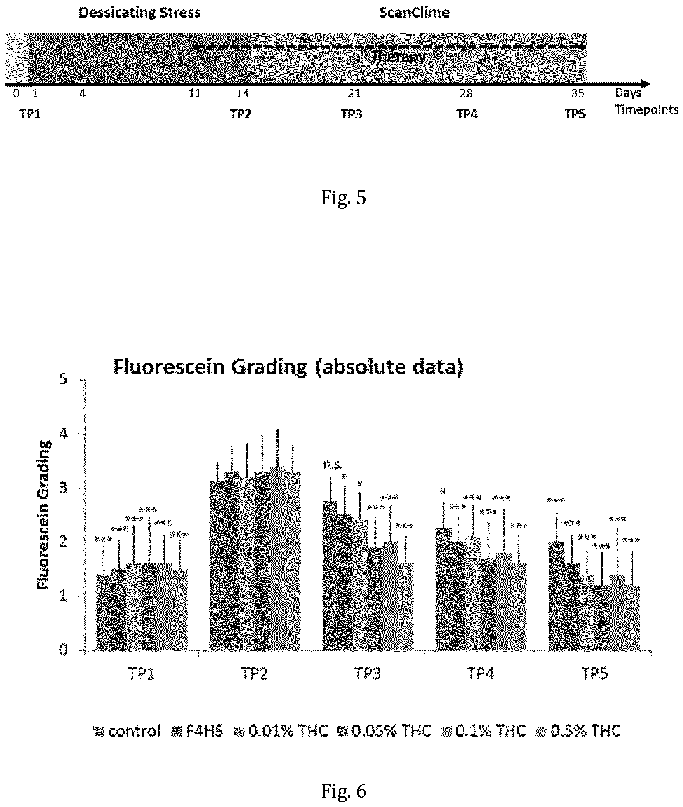

Fig. 5 shows the experimental setup of the desiccating stress dry eye disease model as described by Yeh, S. et al., Invest. Ophthalmol. Vis. Sci. 44: 124-12 and B. Strong, et al. in Cornea Jan 2005;24(1):80-5. Herein, the mice were subjected to desiccating stress from day 1 to day 14. Treatment was administered in form of instillation of 5µl of the respective formulations into the eye (3 times per day) starting at day 11 and continuing to day 35. Tear film production was assessed at TP1 (day 0, healthy mice, before application of desiccating stress), TP2 (day 14, mice at desiccating stress, before therapy), TP3 (day 21, mice after desiccating stress held in standard housing under therapy), TP4 (day 28, mice after desiccating stress in standard housing under therapy) and TP5 (day 35, mice after desiccating stress in standard housing under therapy).

Fig. 6 shows the results of fluorescein grading. The separate sets of columns summarize data obtained at TP1 to TP5 for control (no treatment; left column), F4H5 (1-Perfluorobutyl-pentane, second column from the left), 0.1 mg/ml (0.01 %(w/v)) THC in F4H5 (third column from the left) and 0.5 mg/ml (0.05 %(w/v)) THC in F4H5 (fourth column from the left), 1 mg/ml (0.10 %(w/v)) THC in F4H5 (fifth column from the left) and 5 mg/ml (0.50 %(w/v)) THC in F4H5 (right column). Fluorescein data show that in comparison to the control group the formulations of THC in F4H5 lead to the lowest fluorescein staining levels. (the asterisks refer to the statistical level of significance (p-value) with (*) designating p<0,05, (**) designating p<0,01 and (***) designating p<0,001 when time points TP1, TP3, T4 or TP5 were compared to time point TP2)

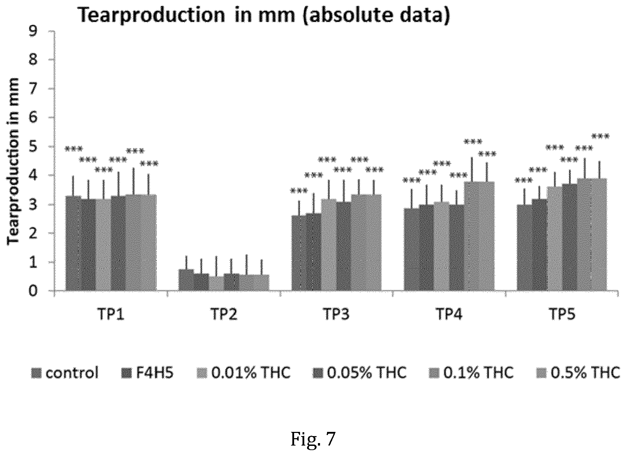

Fig. 7 shows tear production in mm. The separate sets of columns summarize data obtained at TP1 to TP5 for control (no treatment; left column), F4H5 (1-Perfluorobutyl-pentane, second column from the left), 0.1 mg/ml (0.01 %(w/v)) THC in F4H5 (third column from the left) and 0.5 mg/ml (0.05 %(w/v)) THC in F4H5 (fourth column from the left), 1 mg/ml (0.10 %(w/v)) THC in F4H5 (fifth column from the left) and 5 mg/ml (0.50 %(w/v)) THC in F4H5 (right column). The data shows that tear production employing formulations of THC in F4H5 are almost raised back to levels previously recorded for healthy animals at TP1. (the asterisks refer to the statistical level of significance (p-value) with (*) designating p<0,05, (**) designating p<0,01 and (***) designating p<0,001 when time points TP1, TP3, T4 or TP5 were compared to time point TP2)

Fig. 8 shows the results of fluorescein grading. The separate sets of columns summarize data obtained at TP1 to TP5 for control (untreated; left column), treatment with Xiidra® (lifitegrast ophthalmic solution 5% (w/v); 50 mg/ml in buffered aqueous solution) (second column from the left) and treatment with 1 mg/ml (0.1 %(w/v)) THC in F4H5 (right column). The fluorescein grading data show that in comparison to the untreated control group the formulation of 0.1 % (w/v) THC in F4H5 leads to the lowest fluorescein staining levels (statistically significant p-value=0.007 when comparing data at time point TP5). Furthermore, the fluorescein grading data show also that in comparison to the recently FDA-approved DED medication Xiidra® the formulation of 0.1 % (w/v) THC in F4H5 leads to a significant lower fluorescein staining levels (p-value=0.081 when comparing time point TP5). This shows that the treatment with 0.1% (w/v) THC in F4H5 results in an improved healing of ocular surface damage, such as corneal surface damage. On the other hand, when comparing the treatment with Xiidra® to the untreated control group at TP5 a much lower difference was observed, namely with a p-value =0.285.

Fig. 9 shows tear production in mm. The separate sets of columns summarize data obtained at TP1 to TP5 for control (untreated; left column), treatment with Xiidra® (lifitegrast ophthalmic solution 5% (w/v); 50 mg/ml in buffered aqueous solution) (second column from the left) and treatment with 1 mg/ml (0.1 %(w/v)) THC in F4H5 (right column). The data shows that the treatment with a formulation of THC in F4H5 is effective in quickly and sustainably restoring tear production back to levels previously recorded for healthy animals at TP1. Further, it was observed that the treatment with a formulation of THC in F4H5 seems to be more effective in restoring tear production when compared to the treatment with the recently FDA-approved DED medication Xiidra®.

The following examples serve to illustrate the invention.

### EXAMPLES

### Preparation of formulations comprising a cannabinoid receptor binding ligand:

### Preparation of THC-formulations:

A stock solution of delta-9-tetrahydrocannabinol (THC) with a concentration of 1 mg/ml was prepared by dissolving 7,546 mg of Dronabinol (THC Pharm GmbH, Frankfurt, Germany) which was pre-warmed to 55°C for 5 min in 7,55 ml of 1-perfluorobutyl-pentane (F4H5) in glass vial. 5 ml of a THC formulation with a THC content of 0,5 mg/ml were obtained by adding 2,5 ml of F4H5 to 2,5 ml of the THC stock solution (1mg/ml). 5 ml of a THC formulation with a concentration of 0,1 mg/ml were obtained by adding 4,5 ml of F4H5 to 0,5 ml to the THC stock (1mg/ml). The clear solutions comprising 0,1 mg/ml, 0,5 mg/ml or 1 mg/ml THC in 1-perfluorobutyl-pentane were placed in a closed cabinet and stored at room temperature. A stock solution of delta-9-tetrahydrocannabinol (THC) with a concentration of 5 mg/ml was prepared accordingly.

### Preparation of beta-caryophyllene-formulations:

The following stock solutions of β-caryophyllene in F4H5 or F6H8 were prepared:

**Table 1:**

| β-Caryophyllene, dissolved in: | Conc. (mg/ml) |
|---|---|
| F4H5 | 64,23 |
| F6H8 | 95,20 |
| 10 wt% ethanol in F4H5 | 134,62 |
| 10 wt% ethanol in F6H8 | 150, 66 |

### Preparation of cannabidiol (CBD)-formulations:

The following stock solutions of cannabidiol (CBD) in F4H5 or F6H8 were prepared:

**Table 2:**

| Cannabidiol, dissolved in: | Concentration 1 (mg/ml) | Concentration 2 (mg/ml) | Concentration 3 (mg/ml) |
|---|---|---|---|
| F4H5 | 0,60 | | |
| F6H8 | 0,50 | | |
| 0.5 wt% ethanol in F4H5 | 2,03 | 3,81 | 5,18 |
| 2.5 wt% medium chain triglyceride in F4H5 | 1,13 | 2,88 | 4,37 |
| 0.5 wt% ethanol in F6H8 | 1,66 | 2,70 | 4,90 |

### Experimental Dry Eye Model:

Experimental dry eye (EDE) was induced in 10 - 12 weeks old female C57BL/6 mice purchased from Charles River Laboratories Germany GmbH (Sulzfeld, Germany) as described by Yeh, S. et al, Invest. Ophthalmol. Vis. Sci. 44: 124-12 *and* B. Strong, et al. in Cornea Jan 2005;24(1):80-5 according to the timeline as outlined in Fig. 1. The mice were placed in a controlled environment chamber (humidity 30 ± 5 %, constant airflow for 16 hours, temperature 25 ±1 °C) for 14 days. Scopolamine was administered (0,1 mg/day) by subcutaneous implanted osmotic pumps (Alzet®, Charles River Laboratories International, Inc., model #1002). The pumps were explanted after two weeks (day 14). After 14 days of desiccating stress animals were transferred to normal controlled housing conditions (humidity 45-55%, no airflow, temperature 24 ± 2 °C) for further 3 weeks. Climatic changes were hourly logged and checked automatically (KlimaLogg-Pro, TFA Dostmann GmbH & Co. KG, Germany).

All animals were treated according to the German Animal Protection Law (LANUV) and the ARVO statement for the use of animals in ophthalmic research.

### Topical therapy:

### Treatment with compositions comprising THC in F4H5:

Mice were distributed in four groups:
(1) control (left column in Figs. 2 and3)
(2) F4H5 (1-perfluorobutyl-pentane, vehicle) (second column from the left in Figs. 2 and 3)
(3) 0,1 mg/ml (0.01 %(w/v)) THC in F4H5 (third column from the left in Figs. 2 and 3)
(4) 0,5 mg/ml (0.05 %(w/v)) THC in F4H5(right column in Figs. 2 and 3)

The control group (1) was left untreated and received no eye drops, but was housed under the same desiccating stress and standard housing conditions as the three therapy groups. Groups (2) to (4) were treated with 5µl/eye, of 1-perfluorobutyl-pentane (group (2)) or of a 0,1 mg/l solution of THF in F4H4 (group (3)) or of a 0,5 mg/ml solution of THC in F4H5 (group (4)), respectively. The solutions were applied topically to the eye 3 times daily starting from day 11 of the experimental dry eye procedure.

### Treatment with compositions comprising other cannabinoid receptor binding ligands:

The protocol as described above can be followed for compositions comprising a cannabinoid receptor binding ligand such as, for example beta caryophyllene, Cannabidiol (CBD), CP55,940, WIN55,212-2, HU-308, N-arachidonyl glycine and others in a suitable vehicle comprising a semifluorinated alkane, e.g. in solution in F4H5 or F6H8 optionally comprising a cosolvent, such as ethanol. The topical therapy is then conducted with the prepared solutions of a cannabinoid receptor binding ligand in an SFA-containing liquid vehicle as described above in various concentrations (corresponding to groups (3) and (4) above against a control (1) and the chosen liquid vehicle (2).

### Readout Parameters

Clinical signs of dry eye were measured at several time points (TP): TP1: baseline-day 0; TP2: day 14; TP3: day 21; TP4: day 28 as production of tear fluid and corneal damage.

For measurement of tear production phenol red threads (Zone Quick Thread, Oasis® Medical, USA) were placed into the inferior cul-de-sac for 30 seconds and recorded in millimeters.

The extent of corneal damage (and its concomitant healing status) was detected by fluorescein grading. Herein, 5µl of 5% fluorescein in normal saline solution was applied to the eye, carefully wiped off after 30 seconds and graded under blue light using a modified Oxford grading scheme as described in A. J. Bron, et al. Cornea. 2003;22(7):640-50, with severities ranging from grade 0 to grade 5.

At day 28 all mice were sacrificed and their corneas were removed for histological assessment and cervical lymphnodes were collected. For staining of the corneal nerves, whole corneas were excised and fixed in 4% fresh paraformaldehyde over 30 min. at room temperature followed by a permeation step consisting of three washes with 1% Triton X-100 (Triton)/PBS for 15 minutes. Samples were then blocked overnight with 10% normal donkey serum (NDS) in 0.1% Triton/PBS (phosphate buffered saline), followed by overnight incubation with anti-β III tubulin (catalog no. 18207; 1:1000 dilution; Abcam, Cambridge, MA, USA). Incisions were made in each cornea in order to obtain a flower-shaped whole mount (four quadrants) prior to mounting in 50% glycerol for imaging, as described by Chucair-Elliot et al., Invest Ophthalmol Vis Sci. 2015, 56(2), 1097-107 and Bazan et al. NG, Bazan, Exp Eye Res. 2010, 91(4),513-23. Finally, the central and the peripheral area of the cornea were imaged with a fluorescence microscope (Olympus BX53, Hamburg, Germany).

### Results

After termination of desiccating stress and following 7 days of treatment all groups demonstrated a significant increase in tear production at TP3. The results are summarized in Table 3 below; values are given with their standard deviation. Mice in groups (3) and (4) (0.1 mg/ml and 0.5 mg/ml THC in F4H5, respectively) had a significant larger increase in tear production after EDE (Experimental Dry Eye) compared to group (2) and the untreated control group (1) (see Fig. 3).

**Table 3: Results of measurement of tear production**

| **Tear production in mm** | **Baseline** | **DED** | | **Therapy** |
|---|---|---|---|---|
| | **TP1** | **TP2** | **TP3** | **TP4** |
| control | 2,88 ± 0,6 | 0,50 ± 0,5 | 3,38 ± 0,5 | 2,00 ± 0 |
| vehicle (F4H5) | 3,00 ± 0,7 | 0,80 ± 0,6 | 3,30 ± 0,7 | 2,25 ± 0,5 |
| 0.1 mg/ml THC in F4H5 | 3,05 ± 0,7 | 0,65 ± 0,7 | 3,63 ± 0,9 | 3,00 ± 0,9 |
| 0.5 mg/ml THC in F4H5 | 3,05 ± 0,5 | 0,70 ± 0,7 | 4,13 ± 0,6 | 3,50 ± 0,8 |

Analysis of corneal damages following late therapy demonstrated a significantly decrease of the fluorescein grading in the group (3) (0.1 mg/ml THC in F4H5) or group (4) (0.5 mg/ml THC in F4H5) at TP3, and even more pronounced at TP4 (see Fig. 2). The vehicle F4H5 (group (2)) and the untreated control group (1) showed comparable less decrease of the fluorescein staining. The results of the fluorescein grading are summarized in Table 4 below; values are given with their standard deviation.

**Table 4: Results of Fluorescein Grading**

| **Fluorescein Grading** | **Baseline** | **DED** | | **Therapy** |
|---|---|---|---|---|
| | **TP1** | **TP2** | **TP3** | **TP4** |
| control | 1,00 ± 0,5 | 3,13 ± 0,6 | 2,63 ± 0,7 | 2,50 ± 0,7 |
| vehicle (F4H5) | 0,80 ± 0,6 | 3,00 ± 0,7 | 2,30 ± 0,7 | 2,25 ± 0,7 |
| 0.1 mg/ml THC in F4H5 | 0,85 ± 0,5 | 3,15 ± 0,5 | 1,88 ± 0,5 | 1,38 ± 0,5 |
| 0.5 mg/ml THC in F4H5 | 0,85 ± 0,5 | 3,20 ± 0,5 | 1,69 ± 0,6 | 1,13 ± 0,4 |

Comparable beneficial effects when utilizing 0.1 mg/ml, 0.5 mg/ml, 1 mg/ml and 5 mg/ml THC in F4H5 in respect to corneal staining and tear production are shown in Figure 6 and Figure 7, respectively.

Staining of corneal nerves by β-III-tubulin, detecting primarily microtubules in nerves revealed that the occurring loss of nerve fibres (see Fig. 4 A+B) was less at TP4 under treatment with 0.1 mg/ml THC in F4H5 (group 3). At TP4 in the group (4) (receiving 0.5 mg/ml THC in F4H5) no nerve damage was visible (see Fig 4D).

A head-to-head comparison of a composition of the present inventions, namely a composition comprising THC and F4H5 (0.1% (w/v) THC in F4H5, to the recently FDA-approved DED medication Xiidra® is shown in Figures 8 (healing of ocular surface damage by measurement of fluorescein staining) and Figure 9 (restoring of tear production, as measurement of tear production in mm).

### Preparation of further THC-formulations:

### (a) THC 5mg/ml, 100 µg/ml alpha-Tocopherol in F4H5

After prewarming the THC (Dronabinol, THC Pharm GmbH, Frankfurt, Germany) contained in a syringe at 55°C for 5 min, 500 mg of THC was weighted into a glass vial and 10 mg of alpha-Tocopherol were added. Afterwards 100 ml (128.8 g) of 1-perfluorobutyl-pentane (F4H5) was added and the resulting mixture was stirred till complete clear solution was achieved.

### (b) THC 5mg/ml, 330 µg/ml BHA, 170 µg/ml BHT in F4H5

After prewarming the THC (Dronabinol, THC Pharm GmbH, Frankfurt, Germany) contained in a syringe at 55°C for 5 min, 500 mg of THC was weighted into a glass vial and 33 mg of butylated hydroxyanisole (BHA) and 17 mg of butylated hydroxytoluene (BHT) were added. Afterwards 100 ml (128.8 g) of 1-perfluorobutyl-pentane (F4H5) was added and the resulting mixture was stirred till complete clear solution was achieved.

## Claims

1. A pharmaceutical composition comprising:
a) a therapeutically effective amount of at least one cannabinoid receptor binding ligand and
b) a liquid vehicle comprising at least one semifluorinated alkane;
wherein the at least one cannabinoid receptor binding ligand is delta-9-tetrahydrocannabinol (THC) or any pharmaceutically acceptable salt thereof.

2. The pharmaceutical composition according to claim 1, wherein the at least one semifluorinated alkane is a compound of the formula F(CF₂)ₙ(CH₂)ₘH wherein n and m are integers independently selected from the range of 3 to 10.

3. The pharmaceutical composition according to any preceding claim, wherein the at least one semifluorinated alkane is selected from the group consisting of: F4H4, F4H5, F4H6, F4H7, F4H8, F5H4, F5H5, F5H6, F5H7, F5H8, F6H2, F6H4, F6H6, F6H7, F6H8, F6H9, F6H10, F6H12, F8H8, F8H10, F8H12 and F10H10.

4. The pharmaceutical composition according to any preceding claim, wherein the at least one semifluorinated alkane is selected from F4H5 and F6H8.

5. The pharmaceutical composition according to any preceding claim, wherein the composition is in the form of a suspension or in the form of a solution.

6. The pharmaceutical composition according to any preceding claim, wherein the composition is essentially free of water.

7. The pharmaceutical composition according to any preceding claim, wherein the composition further comprises one or more further excipients.

8. The pharmaceutical composition according to any preceding claim, wherein the concentration of the cannabinoid receptor binding ligand in the pharmaceutical composition is in the range from about 0,01 to about 10 mg/ml.

9. The pharmaceutical composition according to any preceding claim, wherein the composition is essentially free of an antioxidant.

10. The pharmaceutical composition according to claim 7, wherein the one or more further excipients comprise an antioxidant.

11. The pharmaceutical composition according to any preceding claim for use as a medicament.

12. The pharmaceutical composition for use according to claim 11, wherein the composition is administered topically to the eye, eye sac, eye surface, the cornea, the and/or to an ophthalmic tissue of a patient.

13. The pharmaceutical composition according to any one of claims 1 to 12, for use in the treatment of neuropathic pain

14. The pharmaceutical composition according to any one of claims 1 to 12, for use in the treatment of keratoconjunctivitis sicca or a symptom or a condition related thereto.

15. A pharmaceutical kit comprising
i. a pharmaceutical composition according to any one of claims 1 to 10 comprising a therapeutically effective amount of at least one cannabinoid receptor binding ligand or any pharmaceutically acceptable salt thereof, and a liquid vehicle comprising at least one semifluorinated alkane;
ii. a container for holding the composition, wherein said container comprises a dispensing means adapted for topical administration of the composition to an eye surface, into a lower eyelid, to the lacrimal sac or to an ophthalmic tissue, and
iii. directions for use of the composition in the therapy, treatment, prevention or amelioration of ophthalmic disorders or diseases.
wherein the at least one cannabinoid receptor binding ligand is delta-9-tetrahydrocannabinol (THC) or any pharmaceutically acceptable salt thereof.

## Patentansprüche

1. Pharmazeutische Zusammensetzung, umfassend:
a) eine therapeutisch wirksame Menge mindestens eines Cannabinoidrezeptor-bindenden Liganden und
b) ein flüssiges Vehikel, das mindestens ein teilfluoriertes Alkan umfasst;
wobei es sich bei dem mindestens einen Cannabinoidrezeptor-bindenden Liganden um delta-9-Tetrahydrocannabinol (THC) oder ein pharmazeutisch unbedenkliches Salz davon handelt.

2. Pharmazeutische Zusammensetzung nach Anspruch 1, wobei es sich bei dem mindestens einen teilfluorierten Alkan um eine Verbindung der Formel F(CF₂)ₙ(CH₂)ₘH handelt, wobei n und m für ganze Zahlen stehen, die unabhängig aus dem Bereich von 3 bis 10 ausgewählt sind.

3. Pharmazeutische Zusammensetzung nach einem der vorhergehenden Ansprüche, wobei das mindestens eine teilfluorierte Alkan aus der Gruppe bestehend aus F4H4, F4H5, F4H6, F4H7, F4H8, F5H4, F5H5, F5H6, F5H7, F5H8, F6H2, F6H4, F6H6, F6H7, F6H8, F6H9, F6H10, F6H12, F8H8, F8H10, F8H12 und F10H10 ausgewählt ist.

4. Pharmazeutische Zusammensetzung nach einem der vorhergehenden Ansprüche, wobei das mindestens eine teilfluorierte Alkan aus F4H5 und F6H8 ausgewählt ist.

5. Pharmazeutische Zusammensetzung nach einem der vorhergehenden Ansprüche, wobei die Zusammensetzung in Form einer Suspension oder in Form einer Lösung vorliegt.

6. Pharmazeutische Zusammensetzung nach einem der vorhergehenden Ansprüche, wobei die Zusammensetzung im Wesentlichen frei von Wasser ist.

7. Pharmazeutische Zusammensetzung nach einem der vorhergehenden Ansprüche, wobei die Zusammensetzung ferner einen oder mehrere weitere Hilfsstoffe umfasst.

8. Pharmazeutische Zusammensetzung nach einem der vorhergehenden Ansprüche, wobei die Konzentration des Cannabinoidrezeptor-bindenden Liganden in der pharmazeutischen Zusammensetzung im Bereich von etwa 0,01 bis etwa 10 mg/ml liegt.

9. Pharmazeutische Zusammensetzung nach einem der vorhergehenden Ansprüche, wobei die Zusammensetzung im Wesentlichen frei von einem Antioxidans ist.

10. Pharmazeutische Zusammensetzung nach Anspruch 7, wobei der oder die mehreren weiteren Hilfsstoffe ein Antioxidans umfassen.

11. Pharmazeutische Zusammensetzung nach einem der vorhergehenden Ansprüche zur Verwendung als Medikament.

12. Pharmazeutische Zusammensetzung zur Verwendung nach Anspruch 11, wobei die Zusammensetzung topisch an das Auge, den Augensack, die Augenoberfläche, die Hornhaut und/oder ein Augengewebe eines Patienten verabreicht wird.

13. Pharmazeutische Zusammensetzung nach einem der Ansprüche 1 bis 12 zur Verwendung bei der Behandlung von neuropathischen Schmerzen.

14. Pharmazeutische Zusammensetzung nach einem der Ansprüche 1 bis 12 zur Verwendung bei der Behandlung von Keratokonjunktivitis sicca oder einem damit zusammenhängenden Symptom oder Leiden.

15. Pharmazeutisches Kit, umfassend
i. eine pharmazeutische Zusammensetzung nach einem der Ansprüche 1 bis 10, umfassend eine therapeutisch wirksame Menge mindestens eines Cannabinoidrezeptor-bindenden Liganden oder eines pharmazeutisch unbedenklichen Salzes davon und ein flüssiges Vehikel, das mindestens ein teilfluoriertes Alkan umfasst;
ii. einen Behälter zur Aufnahme der Zusammensetzung, wobei der Behälter eine Spendeeinrichtung aufweist, die für die topische Verabreichung der Zusammensetzung an eine Augenoberfläche, in ein unteres Augenlid, an den Tränensack oder an ein Augengewebe ausgelegt ist; und
iii. Anweisungen zur Verwendung der Zusammensetzung bei der Therapie, Behandlung, Prävention oder Besserung von Augenleiden oder Augenerkrankungen;
wobei es sich bei dem mindestens einen Cannabinoidrezeptor-bindenden Liganden um delta-9-Tetrahydrocannabinol (THC) oder ein pharmazeutisch unbedenkliches Salz davon handelt.

## Revendications

1. Composition pharmaceutique comprenant :
a) une quantité thérapeutiquement efficace d'au moins un ligand de liaison au récepteur cannabinoïde et
b) un véhicule liquide comprenant au moins un alcane semi-fluoré ;
l'au moins un ligand de liaison au récepteur cannabinoïde étant le delta-9-tétrahydrocannabinol (THC) ou tout sel de celui-ci pharmaceutiquement acceptable.

2. Composition pharmaceutique selon la revendication 1, l'au moins un alcane semi-fluoré étant un composé de formule F(CF₂)ₙ(CH₂)ₘH, n et m étant des nombres entiers choisis indépendamment dans la gamme de 3 à 10.

3. Composition pharmaceutique selon l'une quelconque des revendications précédentes, l'au moins un alcane semi-fluoré étant choisi dans le groupe comprenant : F4H4, F4H5, F4H6, F4H7, F4H8, F5H4, F5H5, F5H6, F5H7, F5H8, F6H2, F6H4, F6H6, F6H7, F6H8, F6H9, F6H10, F6H12, F8H8, F8H10, F8H12 et F10H10.

4. Composition pharmaceutique selon l'une quelconque des revendications précédentes, l'au moins un alcane semi-fluoré étant choisi entre F4H5 et F6H8.

5. Composition pharmaceutique selon l'une quelconque des revendications précédentes, la composition se présentant sous la forme d'une suspension ou sous la forme d'une solution.

6. Composition pharmaceutique selon l'une quelconque des revendications précédentes, la composition étant sensiblement exempte d'eau.

7. Composition pharmaceutique selon l'une quelconque des revendications précédentes, la composition comprenant en outre un ou plusieurs autres excipients.

8. Composition pharmaceutique selon l'une quelconque des revendications précédentes, la concentration du ligand de liaison au récepteur cannabinoïde dans la composition pharmaceutique étant dans la gamme allant d'environ 0,01 à environ 10 mg/ml.

9. Composition pharmaceutique selon l'une quelconque des revendications précédentes, la composition étant sensiblement exempte d'antioxydant.

10. Composition pharmaceutique selon la revendication 7, le ou les autres excipients comprenant un antioxydant.

11. Composition pharmaceutique selon l'une quelconque des revendications précédentes destinée à être utilisée comme médicament.

12. Composition pharmaceutique destinée à être utilisée selon la revendication 11, la composition étant administrée par voie topique dans l'œil, dans le sac oculaire, à la surface de l'œil, sur la cornée, et/ou sur un tissu ophtalmique d'un patient.

13. Composition pharmaceutique selon l'une quelconque des revendications 1 à 12, destinée à être utilisée dans le traitement de douleurs neuropathiques.

14. Composition pharmaceutique selon l'une quelconque des revendications 1 à 12, destinée à être utilisée dans le traitement de la kératoconjonctivite sèche ou d'un symptôme ou d'un état qui est lié à celle-ci.

15. Kit pharmaceutique comprenant
i. une composition pharmaceutique selon l'une quelconque des revendications 1 à 10 comprenant une quantité thérapeutiquement efficace d'au moins un ligand de liaison au récepteur cannabinoïde ou de tout sel de celui-ci pharmaceutiquement acceptable, et un véhicule liquide comprenant au moins un alcane semi-fluoré ;
ii. un récipient destiné à contenir la composition, ledit récipient comprenant un moyen de distribution adapté à l'administration topique de la composition sur une surface oculaire, sur une paupière inférieure, dans le sac lacrymal ou sur un tissu ophtalmique, et
iii. mode d'emploi de la composition dans la thérapie, le traitement, la prévention ou l'amélioration de troubles ou maladies ophtalmiques,
l'au moins un ligand de liaison au récepteur cannabinoïde étant le delta-9-tétrahydrocannabinol (THC] ou tout sel de celui-ci pharmaceutiquement acceptable.
